# EUROPEAN PATENT APPLICATION

(11) **EP 4 459 626 A1**
(43) Date of publication of application: **06.11.2024**
(21) Application number: 23171524.4
(22) Date of filing: 04.05.2023
(51) Int. Cl.: G16H 10/40, G16H 30/40, G16H 50/20, G16H 50/70, C12Q 1/00

(54) **METHOD OF ADJUDICATING AN IMAGED LESION**

(71) Applicant: Siemens Healthineers AG, 91301 Forchheim (DE)
(72) Inventor: HÖLZER, Philipp, Tokyo 141-0021 (JP); HECKEL, Tobias, 81379 München (DE); ASSMANN, Stefan, 91054 Buckenhof (DE); KARABAYIR, Ayse, 90552 Röthenbach (DE); KLATT, Torbjörn, 50374 Erftstadt (DE); GUTSCHE, Robin, 91052 Erlangen (DE); SCHMIDT, Sebastian, 91085 Weisendorf (DE); KAMEN, Ali, 08558 Skillman (US); SINGH, Vivek, 08540 Princeton (US); BROST, Alexander, 91058 Erlangen (DE); SIEBERT, Matthias, 91080 Marloffstein (DE); SPERL, Jonathan, 96047 Bamberg (DE)
(74) Representative: Siemens Healthineers Patent Attorneys

(57) **Abstract**

The invention describes a computer-implemented method of adjudicating an imaged lesion (20), which computer-implemented method comprises receiving a diagnostic image (2) showing a lesion (20); processing the diagnostic image (2) in a machine learning algorithm (1NN) previously trained to classify the imaged lesion (20) and to propose, on the basis of the lesion class (Cx), a blood test panel (3) most suited to adjudicate the imaged lesion (20); and outputting the proposed blood test panel (3) to a user. The invention further describes a method of adjudicating an imaged lesion (20).

## Description

### Background

Various medical conditions can be detected using diagnostic imaging techniques. Particularly in the case of an imaged lesion indicating the presence of a tumor, it is important to be able to correctly assess the nature of the lesion. A false negative diagnosis results in the medical condition going undetected. A false positive diagnosis can be followed by unnecessary and costly invasive procedures. It is known to apply machine learning to assist in identifying imaged lesions, for example a neural network can be trained on many CT images to learn to classify a lesion, primarily on the basis of shape and size. However, the use of the known machine learning methods is largely limited to classification. Therefore, in order to adjudicate a lesion, i.e. to reach a decision as regards the degree of malignancy of the lesion, a clinician must rely on experience and/or avail of further diagnostic tests.

Therefore, in addition to diagnostic imaging, the assessment of a medical condition preferably involves at least one further laboratory diagnostic test. Diagnostic imaging and laboratory diagnostics are fundamentally different in the way they query underlying information. This can be used to good effect in the context of complex clinical questions such as assessing the malignancy of a tumor or lesion, helping a clinician to plan a suitable schedule for subsequent patient management. For example, if a diagnostic image and a blood test both independently suggest malignancy, an invasive procedure such as biopsy or resection can be justified with a high degree of confidence.

However, there is a vast number of established and proprietary blood tests, each configured to detect a certain combination of biological markers present above certain threshold levels in the blood (biological markers present in the blood are generally referred to simply as "blood markers"). Blood tests can differ in their range of validity across various subgroups such as lesion size range, histopathological subtype, patient age, patient smoking history, nodule composition, disease prevalence etc. Blood tests can also differ with regard to the clinical question, i.e. whether the lesion is benign or malignant, whether the lesion is a primary or secondary tumor, whether the lesion is aggressive or indolent, the level of invasiveness of the lesion, whether the lesion is a small-cell or non-small-cell carcinoma, whether the lesion shows druggable molecular alterations, etc. Clearly, it is not practicable to apply every test for every patient.

It is therefore an object of the invention to provide an improved way of combining a diagnostic imaging procedure and a laboratory diagnostic procedure.

This object is achieved by the claimed method of adjudicating an imaged lesion and by the claimed machine learning algorithm.

### Description

According to the invention, the computer-implemented method for use in adjudicating an imaged lesion comprises steps of: receiving a diagnostic image showing a lesion; processing the diagnostic image in a machine learning algorithm trained to assess or classify an imaged lesion and trained to propose, on the basis of lesion class, a blood test panel comprising markers most suited to adjudicate the imaged lesion; and outputting the proposed blood test panel to a user.

In the context of the invention, the proposed blood test panel may represent a single blood test or several blood tests. A clinician can then order one or more blood samples to be obtained and processed according to the proposed blood test panel, and can then adjudicate the lesion from the blood test results. An advantage of the inventive computer-implemented method is that an analysis of image-based features is sufficient for the machine learning algorithm to recommend the most promising blood test panel for that patient in order to reliably adjudicate the lesion, i.e. to answer a specific clinical question such as the degree of malignancy of the lesion. After using the inventive computer-implemented method, it can be sufficient to carry out a single blood test on a panel comprising the set of blood biomarkers that will provide a most informative result to help the clinician obtain an answer to a specific clinical question. This is an improvement over known procedures in which several successive blood tests may be required in order to facilitate satisfactory adjudication of a lesion.

Typically, a lesion is classified as low risk, intermediate risk, or high risk in the context of its malignancy. Depending on the risk group, subsequent patient management can involve surveillance imaging, biopsy, surgery, radiation therapy, ablation etc. as appropriate. However, if a lesion is unambiguously classified in a risk group, ordering of a subsequent blood test is of little clinical benefit. This might be the case for a very large lesion (greater than 30 mm). The clinical impact of a blood test is highest if an imaged lesion cannot be assigned unequivocally to a risk group, i.e. the malignancy risk of the lesion is at the threshold between low risk and intermediate risk, or at the threshold between intermediate risk and high risk. In such threshold cases, the machine learning algorithm can choose blood markers on the basis of their estimated effect size.

In the claimed computer-implemented method, the machine learning algorithm preferably disregards any blood marker with a small effect size and selects only blood markers with large effect sizes. In this way, the machine learning algorithm can propose a blood test panel that is most useful in identifying the correct risk group into which the lesion can be assigned, i.e. the machine learning algorithm provides a way of more accurately adjudicating a lesion.

The machine learning algorithm for use in the inventive computer-implemented method is preferably a multi-class classifier configured to classify an imaged lesion into one of a plurality of predefined classes; and to identify a blood panel which will deliver the most accurate results in a subsequent step of adjudicating the imaged lesion.

Training such a machine learning algorithm preferably comprises the steps of:
A) compiling a training data set comprising an image showing a lesion, a classification of the lesion, and the patient outcome;
B) assigning a ground truth to the training data set, which ground truth comprises a set of biomarkers of a blood panel performed for the respective patient and the blood panel result;
C) applying the machine learning algorithm to the training data set to identify a set of blood panel biomarkers approximating the ground truth; and
repeating steps A - C for a plurality of training data set until a desired level of accuracy has been achieved.

The object of the invention is also achieved by a computer program product with a computer program that is directly loadable into the memory of a data-processing apparatus, and which comprises program units to perform the steps of the inventive computer-implemented method when the program is executed by the control unit.

The machine learning algorithm can be realized as a module of a computer program which can run on any suitable platform and which comprises program units to perform the steps of the inventive computer-implemented method to classify an imaged lesion, to identify blood markers suited to adjudicate the imaged lesion, and to output a blood test panel comprising the identified blood markers. In the context of the invention, a computer program that incorporates the trained machine learning algorithm and has means of receiving relevant input (a diagnostic image and any other relevant inputs as described above can be input to the computer program in any suitable file format) and a means of outputting the proposed blood panel is referred to herein as an "adjudication program" or "adjudication assistant". The computer program can be configured to receive or read a diagnostic image (and any other relevant inputs) in any suitable format and can present the proposed blood test panel to the user in any suitable manner (e.g. to print the result and/or to send it to a further device such as a computer, a mobile device, a central laboratory, etc.). The inventive computer-implemented method can be used by a clinician to assist in stratifying patients into different risk groups with corresponding diagnostic workup.

Particularly advantageous embodiments and features of the invention are given by the dependent claims, as revealed in the following description. Features of different claim categories may be combined as appropriate to give further embodiments not described herein.

In the context of the invention, expressions such as "machine learning algorithm", "neural network", "artificial intelligence tool" and "AI model" shall be understood to be synonyms and may be used interchangeably. The terms "blood test panel", "blood panel" and "test panel" may be used interchangeably.

The imaging modality used to obtain the diagnostic image can be computed tomography (CT), magnetic resonance imaging (MRI), positron emission tomography (PET), single-photon emission computed tomography (SPECT), X-ray imaging, ultrasound imaging, optical coherence tomography (OCT), photoacoustic imaging, etc. In the following, without restricting the invention in any way, it may be assumed that the diagnostic image is a computed tomography image (in the form of a 3D volume or a 2D slice), for example an image can be a process spectrally resolved CT image, a photon-counting CT image; a high-resolution CT image, a contrast-enhanced CT image, a pair of inspiratory and expiratory scans, a gated CT scan, a stationary multi-source CT scan.

The inventive computer-implemented method is applicable to various kinds of lesion, for example the imaged lesion can be any of: a pulmonary lesion, an adrenal lesion, a renal lesion, a hepatic lesion, a pancreatic lesion, a mammary lesion. Any of these can be a tumor, and it is important to reliably determine whether the lesion is benign or malignant. If the lesion is malignant, it is important to reliably assess the degree of malignancy. The invention is particularly suited to assist in adjudicating lesions such as pulmonary nodules, since these can be difficult for a clinician to classify from visual assessment of a diagnostic image, and it can be difficult for a clinician to choose suitable biomarkers to assist in adjudicating an imaged pulmonary nodule.

The machine learning algorithm or "AI model" may include an artificial neural network to apply deep learning techniques. The machine learning algorithm is trained to propose a suitable blood test panel to most accurately adjudicate an imaged lesion. The machine learning algorithm can be implemented as a multi-task classifier which first classifies the imaged lesion into one of several predefined classes, and then identifies the blood test panel that will be best at adjudicating the lesion.

In the following, the machine learning algorithm may be assumed to comprise a neural network such as a convolutional neural network (CNN). Any suitable CNN architecture may be used, for example AlexNet. A supervised training procedure of such a neural network preferably comprises a technique of backward-propagation to find the best set of parameters that will enable the network to infer a ground truth from a diagnostic image, in this case to infer the lesion class of an imaged lesion in its first task, and to infer the most suitable blood test panel in its second task. The training procedure is preferably configured to achieve a favourably low cost function.

The lesion classification stage of the machine learning algorithm preferably influences the internal configuration of the proposed blood test panel, i.e. the operating point along the receiver operating characteristic curve (ROC curve) of the entire test panel, the decision thresholds of individual blood markers, the weights of the individual blood markers, etc.

In addition to the image, the associated blood test for that patient, and the patient outcome (e.g. development of lung cancer and the elapsed time to diagnosis), a training data set preferably also comprises annotated image data. The size, shape and position of a lesion - along with various other factors - are highly relevant to the question of its malignancy. Therefore, in a preferred embodiment of the invention, the training method comprises a step of annotating an image of a training dataset to identify features including any of: lesion size, lesion quantity, lesion composition, lesion location, lesion shape, lesion density, texture features, semantic features, vascular convergence, cavitation, calcification.

A training data set is preferably also augmented by the occurrence and extent of comorbidities such as emphysema, fibrosis, edema, consolidation, opacities, pleural effusion, atelectasis, pulmonary emboli, etc. Further relevant information can be the patient's subcutaneous and visceral fat content, bone mineral density, muscle density, organ volumes, etc. A training data set is preferably also augmented by relevant clinical factors such as patient age, sex, smoking history, history of cancer, familial cancer predisposition, occupation, etc.

Some blood markers are detectable in the bloodstream only if the tumor sheds sufficient material in the first place. For example, freely circulating tumor DNA (ctDNA) is released into the bloodstream through apoptotic or necrotic processes, and predominately in the case of larger tumor sizes. Equally, other systemic markers can show higher diagnostic accuracy for smaller nodule sizes, for example autoantibodies and/or micro-RNA (miRNA). The machine learning algorithm learns to identify which markers are most suitable for including in a blood test panel to adjudicate a lesion on the basis of its class.

The machine learning algorithm is trained to compile a blood test panel comprising any markers such as: carcinoembryonic antigen (CEA), pro-gastrin-releasing-peptide (ProGRP), cytokeratins such as Cyfra 21-1, SCC, neuron specific enolase (NSE), C-reactive protein (CRP), interleukin-6 (IL-6), serum amyloid A1 (SAA1), circulating tumor DNA (ctDNA) (mutations and genetic alterations, methylation patterns, fragmentation patterns); circulating tumor cells (chromosome abnormalities); proteins (autoantibodies, oncofetal antigens, structural proteins, hormones, glycoproteins, storage proteins, enzymes), circulating RNA, micro-RNA (miRNA), metabolites, RNA, sugars, exosomes, extracellular vesicles, etc.

The blood markers are chosen depending on the class to which the lesion has been assigned as well as other factors such as its size. For example, certain blood markers have different sensitivities, so that, if the size of the imaged lesion suggests that it might be outside or near of the range of validity of a certain marker, that marker can be assigned a lower weight in the blood panel. Depending how well the shape characteristics of a lesion correlate with the accuracy of a blood marker, the weight of such a marker can be set accordingly.

The machine learning algorithm preferably applies imaging characteristics such as spiculation and nodule type to configure detection thresholds and risk score weighting factors of markers of a fixed blood test panel. A fixed blood test panel can be a proprietary panel or can have been compiled by a laboratory or a clinician.

For example, a diagnostic image could show a pulmonary nodule located in the periphery of the lung, with solid appearance, homogeneous density and without spiculation: these features are indicative of small cell lung cancer (SCLC). The machine learning algorithm preferably specifies a high threshold for the SCC biomarker in the subsequent laboratory processing step, thus according a relatively high weight to a negative SCC test result (this is because SCLC must have low SCC values). In this way, the sensitivity of the test is increased for SCLC. Since SCLC is the most aggressive form of lung cancer, a high specificity of diagnostic imaging and laboratory blood testing would then accurately indicate a high probability of malignancy for the imaged lesion. The physician and patient can be assured that a subsequent invasive and costly medical procedure is justified.

Since the machine learning algorithm is to be trained to select a set of blood markers that will assist in adjudicating a lesion, each training data set also includes a blood panel and laboratory test results carried out for that patient. For the training data set, a blood panel need not have been scheduled in connection with an imaged lesion, and it is sufficient that the blood test was performed after detection of the lesion by diagnostic imaging. Preferably, each training data set also includes the patient outcome, e.g. whether or not the imaged lesion led to cancer.

The accuracy of a machine learning algorithm depends to a large extent on the number of datasets with which it is trained. In a preferred embodiment of the invention, the machine learning algorithm is trained using at least several hundred training data sets, more preferably several thousand training data sets. Images used to obtain the machine learning algorithm can be obtained from diagnostic imaging modalities used in one or more clinics. Equally, the machine learning algorithm could also be trained with synthetically generated images in order to improve its accuracy in classifying lesions.

The trained machine learning algorithm can then be used by a clinician to assist in adjudicating an imaged lesion. With the inventive computer-implemented method, a suitable diagnostic image is obtained and input to the machine learning algorithm, which returns a proposed blood test panel comprising a set of blood markers chosen to deliver the most conclusive results.

Of course, the machine learning algorithm can continue to learn by including the actual patient outcome some time after adjudication of a lesion using a blood test panel proposed by the machine learning algorithm.

In a preferred embodiment of the invention, the machine learning algorithm may also propose a suitable laboratory technique for analysis of the proposed blood panel. A laboratory analysis technique can be any of: a next-generation sequencing technique, a polymerase chain reaction technique (PCR), a mass spectrometry technique, an immunoassay technique, a fluorescence in-situ hybridization (FISH) technique, an electrochemical sensing technique.

However, even with accurate classification of the lesion and choice of suitable blood markers, the machine learning algorithm may foresee that the proposed blood panel - even though it comprises the most suitable markers - may return an inconclusive result. Therefore, the machine learning algorithm is preferably also trained to also identify one or more further biomarkers for an additional laboratory test. For example, should the machine learning algorithm predict an inconclusive blood panel result, it may propose one or more further biological markers that may then be helpful in adjudicating the classified lesion. Such biological markers may be obtained from a nasal swab, urine, a bronchial swab, saliva, breast milk, etc.

The inventive adjudication program can run on a processing unit connected to an imaging modality in a clinic. Equally, such a computer program can run on a remote server or cloud computing arrangement.

In a preferred embodiment of the invention, the adjudication program comprises a software module configured to automatically order the recommended blood test. In a further preferred embodiment of the invention, the adjudication program comprises a FHIR (Fast Healthcare Interoperability Resources) communication module configured to pre-fetch clinical data from an electronic medical record of the patient as additional input to the machine learning algorithm. In a further preferred embodiment of the invention, the machine learning algorithm is configured to recommend several blood tests to be performed at different stages to facilitate a long-term trend analysis. This can be especially useful if lesion classification is at the threshold between two risk groups.

Other objects and features of the present invention will become apparent from the following detailed descriptions considered in conjunction with the accompanying drawings. It is to be understood, however, that the drawings are designed solely for the purposes of illustration and not as a definition of the limits of the invention.
Figure 1 illustrates the inventive computer-implemented method of adjudicating an imaged lesion;
Figure 2 illustrates a further aspect of the inventive computer-implemented method;
Figure 3 illustrates an exemplary relationship between lesion size and the diagnostic accuracy of biomarkers;
Figure 4 illustrates a further aspect of using biomarkers to adjudicate a lesion;
Figure 5 illustrates a training stage of the machine learning algorithm used by the inventive computer-implemented method;
Figure 6 illustrates an exemplary embodiment of the invention;
Figure 7 shows a conventional approach in adjudicating an imaged lesion.

In the diagrams, like numbers refer to like objects throughout. Objects in the diagrams are not necessarily drawn to scale.

In the following diagrams, the diagnostic image is represented by a pulmonary CT image, and the lesion is represented by a pulmonary nodule. However, as indicated above, the inventive computer-implemented method is applicable to various kinds of lesion, and the images and lesions shown in the drawings are merely exemplary.

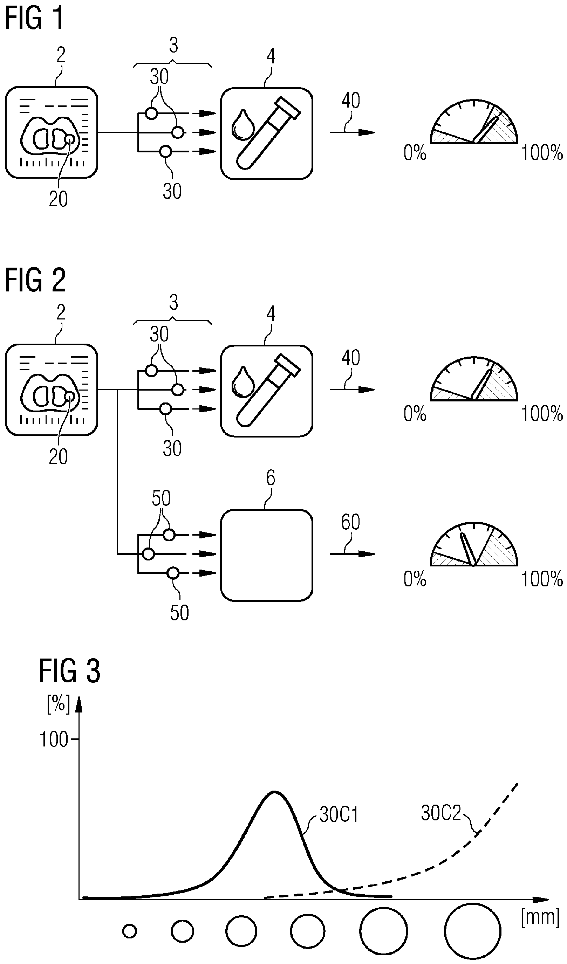

Figure 1 illustrates the inventive computer-implemented method. An image 2 is obtained for a patient, showing a pulmonary nodule 20. The image 2 is processed by the machine learning algorithm 1NN, which outputs a blood test panel 3 comprising the set of blood markers 30 that is most likely to return a conclusive result regarding the malignancy (or not) of the imaged lesion.

A clinician can then order a blood sample to be obtained and sent to a laboratory for processing. The blood sample can be processed according to the blood test panel 3 using any suitable technique such as next-generation sequencing, polymerase chain reaction (PCR), mass spectrometry, enzyme-linked immunosorbent assay (ELISA), fluorescence in-situ hybridization (FISH), etc. The machine learning algorithm 1NN may also recommend a different processing technique as appropriate.

On the right-hand side of the diagram, a dial represents a scale between 0% (lowest risk of malignancy, i.e. the imaged tumor is most likely benign) and 100% (highest risk of malignancy). The machine learning algorithm will have compiled a blood panel which, after laboratory processing of the blood sample 4, will return information allowing the clinician to accurately determine the malignancy of the lesion. The outcome of the adjudication is represented symbolically by the dial indicator. In this example, the indicator is clearly positioned within one of three risk groups.

Figure 2 illustrates a further aspect of the inventive computer-implemented method. Here, the machine learning algorithm 1NN predicts that the proposed blood panel 3 may be inconclusive, and has identified one or more further biomarkers 50 for an additional laboratory test 6. The diagram shows that the results 40 of the blood sample 4 tested according to the blood panel 3 are indeed inconclusive in this case (the "dial indicator" is positioned at a threshold between two risk groups), and that the additional laboratory test 6 delivers a conclusive result 60. In this exemplary embodiment, the lower "dial indicator" is clearly positioned within the intermediate risk group, i.e. the lesion can be reliably adjudicated and the clinician can decide on subsequent procedure with more confidence. More importantly, the patient need not - at this stage - undergo an unnecessary and costly invasive procedure more appropriate to a high-risk tumor.

Figure 3 illustrates an exemplary relationship between lesion size (X-axis) and the diagnostic accuracy (Y-axis) of two exemplary biomarkers. The diagram shows that the diagnostic accuracy curve 30C1 of a first biomarker is highest for midsize lesions and low for small lesions and large lesions, while the diagnostic accuracy curve 30C2 of a second biomarker is high only for large lesions. The machine learning algorithm applies this type of information when putting together a diagnostic blood panel 3 for an imaged lesion 20.

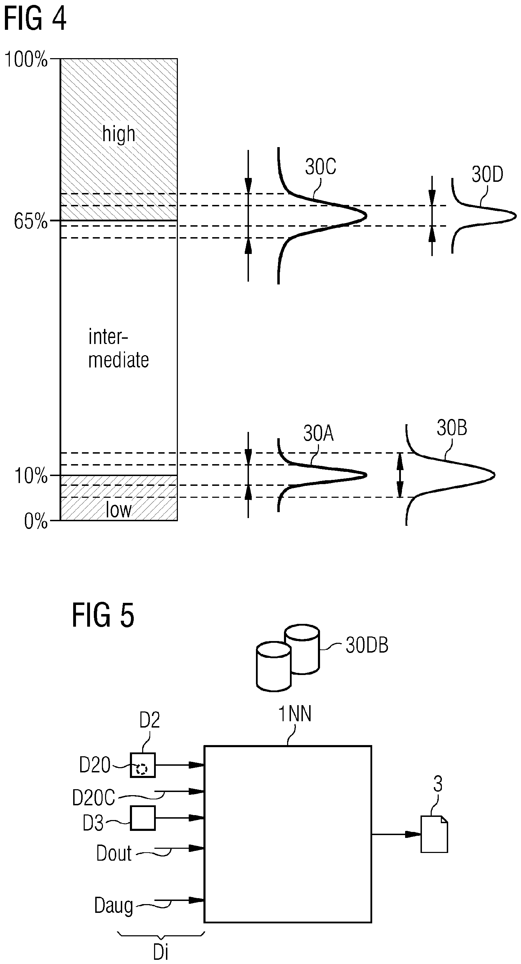

Figure 4 illustrates a further aspect of using biomarkers to adjudicate a lesion. The vertical axis represents a pre-test probability of malignancy. Representing an exemplary situation, the diagram can be understood as follows: if the likelihood of a lesion being malignant is 65% or more, a biopsy or similar is justified; if the likelihood of a lesion being malignant is below 65%, the clinician may order a PET-CT or other follow-up CT and/or a less invasive procedure such as a percutaneous biopsy); if the likelihood of a lesion being malignant is less than 10%, the clinician may opt for surveillance of the patient. However, not all biomarkers are suited to obtaining a conclusive probability of malignancy. For example, biomarker 30A (represented by a diagnostic accuracy curve) is unsuited for adjudicating between a low probability of malignancy and an intermediate probability of malignancy (its effect size is small), while biomarker 30B is better suited (its effect size is large); similarly, biomarker 30C is suited for adjudicating between a low probability of malignancy and an intermediate probability of malignancy, while biomarker 30D is unsuited. The machine learning algorithm 1NN applies this type of information when putting together a set of biomarkers 30 for a diagnostic blood panel 3.

Figure 5 illustrates a training stage of the machine learning algorithm 1NN. Here, training data sets are input to the machine learning algorithm 1NN. Each training data set Di comprises an image D2 showing a lesion D20, the lesion class D20C, the blood panel D3 performed for that patient, and the established malignancy of the imaged lesion, i.e. the patient outcome Dout. Each dataset Di can be augmented with other relevant information Daug such as co-morbidities of the patient, the patient's EHR, etc.

The machine learning algorithm 1NN is trained with at least 100 such training data sets Di. More preferably however, the machine learning algorithm 1NN is trained with several thousand training data sets Di. Training is deemed complete when the machine learning algorithm 1NN is able to classify an imaged lesion with a high degree of accuracy and is able to select a set of most suitable blood markers from an extensive blood marker database 30DB, presenting this as a proposed blood panel 3 to assist in reliably adjudicating the imaged lesion.

The blood marker database 330DB is not restricted to the blood markers appearing in the blood panels D3 of the training data sets Di, but can include further blood markers also, for example, markers chosen on the basis of patient outcome data, biopsy data or follow-up imaging data.

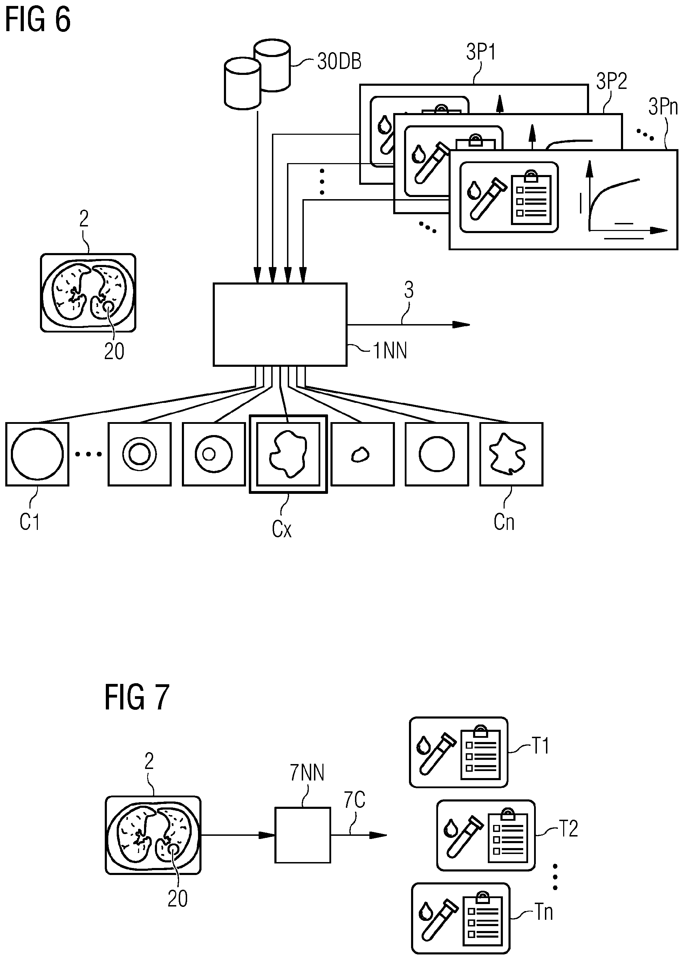

Figure 6 illustrates an exemplary embodiment of the invention. Here, an image 2 showing a lesion 20 is input to the machine learning algorithm 1NN. In a first stage, the machine learning algorithm 1NN classifies the lesion 20 into one of a predetermined set of lesion classes C1, ..., Cn. In this example, the machine learning algorithm 1NN has classified the lesion 20 as belonging to class Cx.

In a subsequent stage, the machine learning algorithm 1NN identifies a blood test panel (with one or more blood markers) that will return the most conclusive information regarding the lesion 20. The machine learning algorithm 1NN can compile a blood panel from a plurality of individual blood markers whose specifics are stored in a database 30DB. In this exemplary embodiment, the machine learning algorithm 1NN can also choose from several predefined blood panels 3P1, ..., 3Pn (e.g. proprietary blood panels), each with a previously established set of blood markers and linked to a predefined diagnostic technique. In a final step, the machine learning algorithm 1NN outputs the proposed blood panel 3 to a user.

Figure 7 shows a conventional approach. Here, a pulmonary CT image 2 is obtained for a patient, showing a pulmonary nodule 20. The image 2 is processed by a machine learning algorithm 7NN which has been trained to classify the lesion. For some types and size of lesion, classification can be done to a high degree of accuracy, allowing a clinician to adjudicate the lesion 20 as benign or malignant with some degree confidence, based on the lesion class 7C returned by the machine learning algorithm 7NN. However, not all lesions can be reliably adjudicated. In an attempt to obtain a reliable diagnosis for the patient, the clinician may then order blood tests T1, ..., Tn on various different biomarkers. Several tests are generally required, because the known blood tests generally have either a high negative predictive or a high positive predictive value, but rarely both. A single blood test chosen on the basis of the lesion class 7C determined using the prior art approach is therefore usually only of limited use in the adjudication of a lesion. The results of a single blood test are often inconclusive, and the clinician may resort to an invasive procedure in order to obtain certainty for the patient. This can add significantly to the overall cost of treatment and can cause undue stress to the patient.

Although the present invention has been disclosed in the form of preferred embodiments and variations thereon, it will be understood that numerous additional modifications and variations could be made thereto without departing from the scope of the invention.

For the sake of clarity, it is to be understood that the use of "a" or "an" throughout this application does not exclude a plurality, and "comprising" does not exclude other steps or elements. The mention of a "unit" or a "module" does not preclude the use of more than one unit or module.

Independent of the grammatical term usage, individuals with male, female or other gender identities are included within the term.

## Claims

1. A computer-implemented method for use in adjudicating an imaged lesion (20), which method comprises
- obtaining a diagnostic image (2) showing a lesion (20);
- inputting the diagnostic image (2) to a machine learning algorithm (1NN) previously trained to classify the imaged lesion (20) and to propose, on the basis of the lesion class (Cx), a blood test panel (3) comprising markers (30) most suited to adjudicate the imaged lesion (20); and
- outputting the proposed blood test panel (3) to a user.

2. A computer-implemented method according to the preceding claim, wherein the blood test panel (3) comprises markers (30) from the group of: circulating tumor DNA, circulating tumor cells, proteins, circulating RNA, metabolites, sugars, exosomes.

3. A computer-implemented method according to any of the preceding claims, wherein the machine learning algorithm (1NN) is trained to estimate the malignancy risk of the imaged lesion (20), and to include at least one marker (30) with a large effect size in the blood test panel (3) when the estimated malignancy risk is at a threshold between two risk groups.

4. A computer-implemented method according to any of the preceding claims, wherein the blood test panel (3) includes one or more of: an operating point along a receiver operating characteristic curve of the blood test panel (3), a decision threshold of a blood marker (30), a weight of a blood marker (30) .

5. A computer-implemented method according to any of the preceding claims, wherein the machine learning algorithm (1NN) is trained to identify at least one laboratory analysis technique for analysis of the proposed blood test panel (3).

6. A computer-implemented method according to the preceding claim, wherein a laboratory analysis technique is any of: a next generation sequencing technique, a polymerase chain reaction technique, a mass spectrometry technique, an immunoassay technique, a fluorescence in-situ hybridization technique, an electrochemical sensing technique.

7. A computer-implemented method according to any of the preceding claims, wherein the machine learning algorithm (1NN) is trained to identify a number of further biological markers (50) to assist in adjudicating the imaged lesion (20) .

8. A computer-implemented method according to the preceding claim, wherein a further biological marker (50) is obtainable from any of: a nasal swab, urine, a bronchial swab, saliva.

9. A computer-implemented method according to any of the preceding claims, wherein the diagnostic image is a computed tomography image (2).

10. A computer-implemented method according to any of the preceding claims, wherein the imaged lesion is any of: a pulmonary lesion (20), an adrenal lesion, a renal lesion, a hepatic lesion, a pancreatic lesion, a mammary lesion.

11. A computer-implemented method according to any of the preceding claims, wherein the machine learning algorithm (1NN) is a two-step classifier configured
- to classify an imaged lesion (20) into one of a plurality of predefined classes (C1, ..., Cn);
- to identify a number of blood markers (30) suited to adjudicate the imaged lesion (20); and
- to output a blood test panel (3) comprising the identified blood markers (30).

12. A method of adjudicating an imaged lesion (20), which method comprises the steps of
- obtaining a diagnostic image (2) showing a lesion (20);
- processing the diagnostic image (2) using the computer-implemented method according to any of claims 1 to 11 and receiving the proposed blood test panel (3); and
- obtaining a blood sample (4) and performing laboratory processing of the blood sample (4) according to the blood test panel (3).

13. A data processing apparatus adapted to perform the steps of the method according to any of claims 1 to 11, which data processing apparatus comprises
- an input interface for obtaining a diagnostic image (2);
- a machine learning algorithm (1NN) previously trained to classify an imaged lesion (20) and to specify, on the basis of the lesion class (Cx), a blood test panel (3) most suited to adjudicate the imaged lesion (20); and
- a means of outputting the proposed blood test panel (3) to a user.

14. A computer program product comprising instructions which, when the program is executed by a computer, cause the computer to carry out the steps of the method according to any of claims 1 to 11.

15. A computer-readable data carrier having stored thereon the computer program product of claim 14.
